(19) Europäisches Patentamt
European Patent Office
Office européen des brevets

(11) EP 3 321 621 A1

(12) # EUROPÄISCHE PATENTANMELDUNG

(43) Veröffentlichungstag:
**16.05.2018 Patentblatt 2018/20**

(51) Int Cl.:
***F27B 17/02*** *(2006.01)*

(21) Anmeldenummer: **16198859.7**

(22) Anmeldetag: **15.11.2016**

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**
Benannte Validierungsstaaten:
**MA MD**

(71) Anmelder: **Ivoclar Vivadent AG**
**9494 Schaan (LI)**

(72) Erfinder:
- **JUSSEL, Rudolf**
**6805 Feldkirch-Gisingen (AT)**
- **BÜRKE, Haral**
**6820 Frastanz (AT)**

(74) Vertreter: **Baronetzky, Klaus**
**Splanemann**
**Patentanwälte Partnerschaft**
**Rumfordstrasse 7**
**80469 München (DE)**

(54) **ANORDNUNG EINES OFENS UND EINES HAUFWERKS VON GLASPARTIKELN SOWIE VERFAHREN ZUM BETRIEBS EINES OFENS**

(57) Die Erfindung betrifft eine Anordnung eines Ofens und eines Haufwerks von Glaspartikeln mit einem Haufwerk von Glaspartikeln (32) und einem Ofen (10), welcher einen Pressstempel (36), einen Druck-, Weg- und/oder Geschwindigkeits-Sensor und eine Steuervorrichtung für die Steuerung eines Pressvorgangs basierend auf dem Ausgangssignal des Sensors aufweist. Der Sensor erfasst mindestens einen Druck-, Orts-, und/oder Bewegungsparameter des Pressstempels (36). Der Pressstempel (36) wirkt - gegebenenfalls über einen zwischengeschalteten Presskolben (28) - auf das Haufwerk von Glaspartikeln (32), die in einem Presskanal (30) geführt und insbesondere kristallisierbar sind. Das Auslösekriterium für die Prozesssteuerung ist eine über den Sensor erfasste Änderung mindestens eines Bewegungsparameters des Pressstempels (36) bei Erweichung des Haufwerks von Glaspartikeln (32).

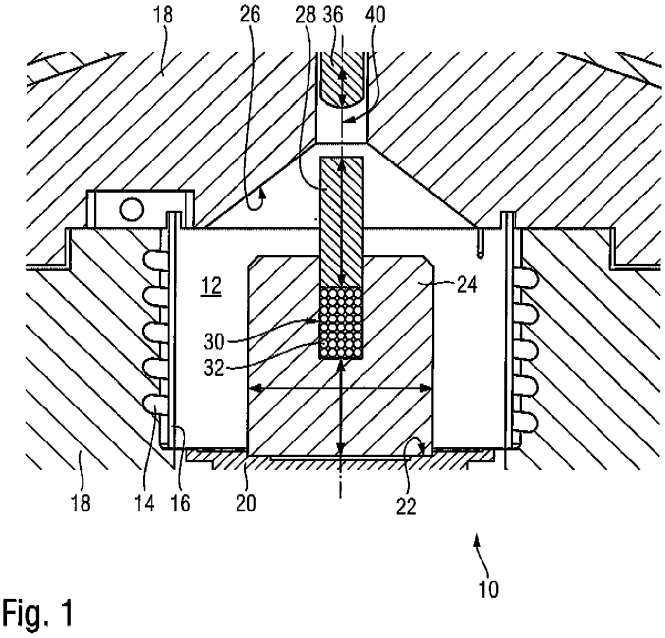


Fig. 1

EP 3 321 621 A1

**Beschreibung**

Die Erfindung betrifft eine Anordnung eines Ofens und eines Haufwerks von Glaspartikeln, gemäß dem Oberbegriff von Anspruch 1, sowie ein Verfahren zum Betrieb eines Ofens, gemäß dem Oberbegriff von Anspruch 12.

Spezielle Öfen wie dentale Brennöfen oder dentale Pressöfen können zur Herstellung von Dentalrestaurationen aus z.B. Keramik oder Glas verwendet werden.

Bei der Herstellung von Dentalrestaurationen aus Glas können Glaskeramik-Pulver und/oder kristallisierbare Glaspartikel zum Einsatz kommen, die in an sich bekannter Weise in einer Muffel eingesetzt werden, welche, vorzugsweise vorab vorgewärmt, in einer Brennkammer eines Brennofens oder in einer Presskammer eines Pressofens auf einem Brennteller platziert wird.

Die Glaskeramik-Pulver bzw. Glaspartikel werden zunächst bis zu einem gewünschten viskosen Zustand erwärmt. Abschließend werden bei einem Verdichtungsprozess diese weichen Glaspartikel unter Druck zu einem festen Block oder über die unter den Glaspartikeln angeordneten Anstiftkanäle der Muffel in die Formräume für Dentalrestaurationsteile gepresst, bis die Formräume vollgefüllt sind.

Durch ein weiteres Entbinderungsverfahren und einen weiteren Abkühlprozess werden Dentalrestaurationsteile aus z.B. Lithiumsilikat bereitgestellt.

Jedoch besteht bei der Verdichtung der Glaspartikel das Problem darin, dass häufig Kristallisation auftritt, bevor die Glaspartikel vollständig verdichtet sind. Diese unkontrollierte Kristallisation behindert die weitere Verdichtung und führt aufgrund eines hohen Anteils an verbleibender Porosität zu einem Festigkeitsverlust.

Außerdem ist die sich dadurch ergebene Glaskeramik minderwertig, weil durch die unkontrollierte Kristallisation ein ungünstiges Kritallgefüge mit breiter Kristallgrößenverteilung entsteht.

Für einen erfolgreichen Pressvorgang von Dentalglaspartikeln ist es ferner wichtig, das Aufheizen mit einem exakt definierten Aufheizprofil vorzunehmen.

Während die gemessenen Temperaturen typischerweise die Ofeninnenraumtemperatur betreffen und insofern nicht die echte Temperatur der Glaspartikel, ist aus der WO 2014/131588 A1 ein Verfahren bekannt geworden, die Temperatur eines Rohlings selbst zu erfassen.

Bei einem großen Haufwerk von Glaspartikeln besteht aber innerhalb des Haufwerks ein gewisser Temperaturgradient, und auch mit der genannten günstigen, aber auch nicht ganz unaufwändigen Lösung lässt sich typischerweise die Temperatur des Haufwerks an einer Stelle, jedoch nicht an allen Stellen erfassen.

Ein weiteres Problem besteht darin, dass bei besonders ausgebildeten Temperaturprofilen die Totzeit des Systems Muffel/Glaspartikel die gemessenen Temperaturprofile verzögert. Diese hängt stark von der Masse des Haufwerks von Glaspartikeln und der Muffel ab. Besonders voluminöse Muffeln und ein großes Haufwerk von Glaspartikeln müssten daher im Grunde mit noch "schärferen" - also längeren und exakten - Aufheizprofilen betrieben werden.

Typischerweise wird nach der Aufheizung eine Haltezeit eingeschoben, die dem Temperaturausgleich dienen soll und in welcher die heißen Außenbereiche der Muffel ihre Wärme an das Muffelzentrum und damit das Haufwerk von Glaspartikeln abgeben.

Diese Haltezeit verlängert jedoch je nach Muffelgröße den Presszyklus beträchtlich, was teilweise nicht als akzeptabel angesehen wird. Zudem muss je nach verwendeter Muffelgröße ein entsprechendes Steuerprogramm mit passenden Temperaturprofilen und Haltezeiten ausgewählt werden. Auch kann durch die Verlängerung der Haltezeit nicht ausgeschlossen werden, dass vorgängig im Prozess relevante Randbedingungen nicht doch auf das Ergebnis durchschlagen.

Daher liegt der Erfindung die Aufgabe zugrunde, eine Anordnung eines Ofens und eines Haufwerks von Glaspartikeln gemäß dem Oberbegriff von Anspruch 1 sowie ein Verfahren zum Betrieb eines Ofen gemäß Oberbegriff von Anspruch 12 zu schaffen, die die Qualität von zu herstellenden Dentalrestaurationsteilen aus Glas sicherstellt und unabhängig von der Größe eines Haufwerks von Glaspartikeln beziehungsweise der zugehörigen Muffel einen verkürzten Presszyklus erlaubt.

Diese Aufgabe wird erfindungsgemäß durch die Ansprüche 1 und 12 gelöst. Vorteilhafte Weiterbildungen ergeben sich aus den Unteransprüchen.

Erfindungsgemäß wird vor der Verdichtung des Haufwerks von Glaspartikeln eine schnelle Aufheizung dieser vorgenommen.

Die Keimbildung der kristallisierbaren Glaspartikel beginnt je nach Material bei dem Transformationsbereich Tg (auch als "Transformationspunkt Tg" bezeichnet) der Glaspartikel und hat während der Zunahme der Temperatur ihre maximale Geschwindigkeit bei einem Temperaturbereich etwa 50 °C über den Transformationsbereich Tg der Glaspartikel (Tg + 50 °C). Bei der weiteren Zunahme der Temperatur verlangsamt sich die Geschwindigkeit der Keimbildung. Die Verdichtungsgeschwindigkeit der Glaspartikel wird in einem Temperaturbereich z.B. zwischen 50 °C über den Transformationsbereich Tg der Glaspartikel (Tg + 50 °C) und 100 °C über den Transformationsbereich Tg der Glaspartikel (Tg + 100 °C) bei der Zunahme der Temperatur erhöht.

In diesem Zusammenhang sind erfindungsgemäß durch eine schnelle Aufheizung der Glaspartikel von etwa dem Transformationsbereich Tg der Glaspartikel auf einen Temperaturbereich z.B. 70 °C über den Transformationsbereich (Tg + 70 °C) lediglich wenige Keime (in gewünschtem Maße) gebildet. Es ist günstig, dass sich bei der Verdichtung gemäß dem Beispiel ab den Temperaturbereich - 70 °C über den Transformationsbereich Tg (Tg + 70 °C) - aufgrund der wenigen Keime auch wenige Glaskristalle ergeben. Dadurch wird gewährleistet, dass die weitere Verdichtung nicht mehr durch die ungewünschte und unkontrollierte Kristallisation (stark) verhindert wird.

Damit lassen sich überraschend die Parameter für den Verdichtungsprozess unabhängig von der Menge der Glaspartikel, dem Material der Glaspartikel sowie der Muffelgröße bestimmen.

In einer bevorzugten Ausführungsform ist es vorgesehen, dass nach dem Verdichtungsvorgang sich das verdichtete Haufwerk der Glaspartikel sofort abkühlen lässt. Denn bei einer weiteren Zunahme der Temperatur - anschließend an das vorstehend genannte Beispiel - über den "Tg + 100 °C"-Bereich verlangsamt sich die Geschwindigkeit der Verdichtung der Glaspartikel, während sich die Geschwindigkeit der Kristallisation beschleunigt. In diesem Fall wird erfindungsgemäß durch eine kontrollierte Temperatur bzw. das nach der Verdichtung sofortige Abkühlen der Glaspartikel das Kristallwachstum weiter gemindert.

Erfindungsgemäß besonders günstig ist es, dass nun mit der Erfindung erstmals die inherenten physikalischen Parameter von Glaspartikeln als Kriterium eingesetzt werden können. Dies stellt einen signifikanten Fortschritt gegenüber den typischen und bislang weit verbreiteten Brennöfen und Pressöfen, bei denen lediglich ein Temperatursensor im Ofeninnenraum zum Einsatz gelangt. Bei der demgegenüber besseren Lösung gemäß der WO 2014/131588 A1 wird immerhin die Oberfläche des zu erzeugenden Dentalrestaurationsteils geprüft. Jedoch besteht gerade bei einem großen Haufwerk von dentalen Glaspartikeln ein Temperaturgradient, und auch die Größe des Haufwerks ist für die Temperaturdifferenz zwischen Innen und Außen verantwortlich. Ein weiterer Nachteil der vorbekannten Lösung ist, dass zusätzliche und kostspielige Messtechnik am Gerät angebracht werden muss.

Typischerweise besteht in einer Muffel ein Temperaturgradient, der sich mit seinen Isothermen, in dreidimensioneler Sicht betrachtet, im wesentlichen glockenförmig erstreckt, wobei - bezogen auf das Haufwerk von Glaspartikeln - unten in der Mitte des Haufwerks die geringste und oben/außen an dem Haufwerk die höchste Temperatur besteht. Erfindungsgemäß werden nun Glaspartikelparameter an den Seitenflächen des Haufwerks als Auslösekriterium für die Prozesssteuerung herangezogen, in einer überraschend raffinierten Weise, wobei die Erweichung der Glaspartikel in ihrer Gesamtheit ausgewertet werden.

Zur Prozesssteuerung gehört der Start des Pressvorgangs, bei welchem Start die Ofentemperatur, der Druck in einem Brennraum des Ofens und eine Presskraft des Pressstempels durch die Steuervorrichtung gesteuert werden. Außerdem gehört dazu das Ende des Pressvorgangs bzw. der Anfang eines an den Pressvorgang anschließenden Abkühlprozesses, bei welchem Ende bzw. Anfang die Presskraft des Pressstempels durch die Steuervorrichtung, insbesondere auf Null, reduziert und die Ofentemperatur durch die Steuervorrichtung, insbesondere mittels der Abschaltung des Ofens und/oder der Öffnung der Ofenhaube, reduziert wird.

Beispielsweise kann der Rückgang der Pressgeschwindigkeit als Auslösekriterium für die Abschaltung des Ofens verwendet werden. Beträgt bei dem Pressvorgang beim Rückgang der Pressgeschwindigkeit, welche durch den Sensor erfasst wird, diese einen Wert nahe Null oder einen Wert weniger als 5 %, insbesondere weniger als 2 %, der maximalen Pressgeschwindigkeit des Pressstempels, ist das Kriterium für die Abschaltung des Ofens als erfüllt anzusehen, was bedeutet, dass automatisch oder manuell der Pressvorgang beendet und der Abkühlprozess gestartet werden sollte.

Außerdem kann der Gegendruck auf den Pressstempel aufgrund der Erwärmung der Glaspartikel und der Einbettmasse im unteren Teil durch einen Drucksensor gemessen und als Auslösekriterium für die Prozesssteuerung verwendet werden. Beispielsweise sollte der Pressvorgang gestartet werden, wenn der erfasste Gegendruck innerhalb einer vorgegebenen Zeit auf eine vorgegebene Höhe konstant bleibt.

Darüber hinaus kann der Ort des Pressstempel durch einen Ortssensor erfasst und als Auslösekriterium für die Prozesssteuerung verwendet werden. Beispielsweise sollte der Ofen abgeschaltet werden, wenn sich der Ort des Pressstempel beim Pressvorgang innerhalb einer vorgegebenen Zeit nahezu nicht ändert.

Vor der Aufheizung wird das erfindungsgemäße Haufwerk von Glaspartikeln aus unterschiedlichen Ausgangsgläsern zunächst in den Presskanal des Ofens eingefüllt und bildet dort Gradienten, insbesondere Farb- und/oder Transluzenzgradienten, damit die herzustellenden Dentalrestaurationsteile gewünschte Gradienten aufweisen, die zur Mundsituation eines Patienten passen.

Die Gradienten entsprechen mindestens einer graduellen schichtweisen und/oder einer kontinuierlichen Änderung einer physikalischen Eigenschaft des Haufwerks von Glaspartikeln.

In einer weiteren bevorzugten Ausführungsform besteht das Haufwerk der Glaspartikel aus verschiedenen eingefärbten Glaspartikeln und/oder mindestens einer Glassorte, die mit Pigmenten unterschiedlich eingefärbt wird. Zu Pigmenten gehören unter anderem Buntpigmente, Trübpigmente und Fluoreszenzpigmente.

Die Verwendung von Pigmenten ist besonders bei vergleichsweise tiefen Prozesstemperaturen geeignet. Bei höheren Prozesstemperaturen sind solche Pigmente chemisch oder thermisch nicht in der jeweiligen Glasmatrix stabil und würden sich teilweise oder vollständig zersetzen.

In einer weiteren bevorzugten Ausführungsform besteht das Haufwerk der Glaspartikel aus Gläsern mit unterschiedlichen Gehalten an Keimbildnern. Als Ergebnis kann nach einer gesteuerten Kristallisation ein Block mit unterschiedlichen Kristallgrößen und/oder unterschiedlichen Festigkeiten in verschiedenen Bereichen resultieren. Beispielsweise kann bei einer hergestellten Dentalrestauration im Dentinbereich eine größere Kristallgröße mit einem weicherem Material mit geringer Transluzenz und im Schmelzbereich ein feinkristallines Gefüge mit hoher Härte, hoher Verschleißfestigkeit und hoher Transluzenz realisiert werden.

In einer weiteren bevorzugten Ausführungsform besteht das Haufwerk der Glaspartikel aus Gläsern mit unterschiedlichen Dichten. Ein besonders weicher, elastischer Bereich, welcher geeignet für die Nachbildung von Dentin mit geringen Härte ist, ist durch geregelte Erzeugung von Restporosität möglich.

Erfindungsgemäß werden auch nicht ideale Startbedingungen aus vorangegangenen Prozessschritten wie eine korrekte Vorwärmetemperatur oder Vorwärmedauer der Muffel berücksichtigt respektive korrigiert bzw. ausgeglichen.

Das Haufwerk von Glaspartikeln wird - lange vor dem Pressvorgang - über den Pressstempel - typischerweise über Zwischenschaltung eines Presskolbens, beispielsweise aus $Al_2O_3$, unter Druck gesetzt. Auf seine obere Stirnfläche wirkt nun eine Kraft und ein Druck, der von seiner Unterseite aufgefangen wird, teilweise aber auch zu einer elastischen Verformung der Glaspartikel - allerdings in (ganz) geringem Maße - führt. Diese Kraft und die Verformung ist jedenfalls ausreichend, die Anordnung der Glaspartikel, welche nach der Einsetzung in der Muffel locker ist, fester und stabiler zu veranlassen.

Die Muffelmasse hat typischerweise einen vergleichsweise geringen Wärmeausdehnungkoeffizienten von beispielsweise $3 \times 10^{-6}$/K, während die Glaspartikel aus z.B. Lithiumsilikat einen deutlich höheren Wärmeausdehnungskoeffizienten von beispielsweise $10 \times 10^{-6}$/K aufweisen.

Glaspartikel haben bis unterhalb des sogenannten Glasübergangsbereichs einen Wärmeausdehnungskoeffizienten von beispielsweise 10 oder $10{,}5 \times 10^{-6}$/K. Der Wärmeausdehnungskoeffizient steigt zwar dann deutlich an, wenn der sogenannte Phasenübergang zweiter Ordnung überschritten ist, beispielsweise auf $14{,}5 \times 10^{-6}$/K. Aufgrund der Erweichung der Glaspartikel ändert sich jedoch insbesondere auch die Form der Glaspartikel. Insbesondere beginnt das Haufwerk der Glaspartikel aber auch in die Anstiftkanäle für die Dentalrestaurations-Hohlräume einzutreten, nachdem das Haufwerk der Glaspartikel nunmehr viskos geworden ist. Dies stellt eine erhebliche Überkompensation des zunehmenden Wärmeausdehnungskoeffizienten dar.

Betrachtet man in diesem Zusammenhang die Parameter der Bewegung des Pressstempels, sind Mikroimpulse festzustellen, die aufgrund der Wärmeausdehnung des Pressstempels, des Presskolbens, der Glaspartikel und der umgebenen Muffel eine geringe und kurzzeitige Bewegung zwischen dem oberen oder stempelnahen Ende des Haufwerks der Glaspartikels und der Muffel wiedergeben. Besonders günstig ist es, dass dadurch der zeitlichen Verlauf der Wärmeausdehnung in Folge der Erwärmung des eingebrachten Systems bestehend aus Muffel, zwischengeschaltetem Presskolben und insbesondere dem Haufwerk der Glaspartikel erfaßt werden kann.

In weiterer vorteilhafter Ausgestaltung ist es vorgesehen, dass die Steuervorrichtung den Pressbeginn unabhängig von der von dem Temperatursensor gemessenen Temperatur in Abhängigkeit der Beendigung der Mikroimpulse festlegt.

In weiterer vorteilhafter Ausgestaltung ist es vorgesehen, dass die Steuervorrichtung den Pressbeginn basierend auf einer Erhöhung der gemessenen Zeitdauer zwischen zwei aufeinanderfolgenden Mikroimpulsen über einen vorgegebenen Schwellwert startet.

In weiterer vorteilhafter Ausgestaltung ist es vorgesehen, dass die Steuervorrichtung bei Zunahme der Zeitdauer zwischen zwei aufeinanderfolgenden Mikroimpulsen über einen vorgegebenen, insbesondere zweiten Schwellwert, die Heizleistung auf einen Nennwert absenkt.

Erfindungsgemäß wird das Zeitprofil der Mikroimpulse ausgewertet und entweder bei Beendigung der Mikroimpulse der Bewegungsparameter der Glaspartikel und/oder bei Erfassung einer vorgegebenen Anzahl bei Mikroimpulsen und/oder durch Auswertung des zeitlichen Abstands zwischen den Mikroimpulsen ein Auslösekriterium für die Prozesssteuerung festgelegt.

Mit der Erfindung lässt sich so z.B. der optimale Pressbeginn bestimmen und festlegen und damit die für den Presszyklus insgesamt aufgewendete Zeit nahe an das Optimum heranführen.

Erfindungsgemäß wird - von den Bewegungsparametern her betrachtet - als Auslösekriterium für den tatsächlichen Pressvorgang das Auftreten von kurzzeitigen Mikrobewegungen überwacht und als Kriterium herangezogen. Es versteht sich, dass die Mikrobewegungen gegen die Pressrichtung gerichtet sind, aufgrund der Wärmeausdehnung des Gesamtsystems Pressstempel/Presskolben/Glaspartikel/ Muffel/Brennkammermantel.

Der Pressstempel bewegt sich während des Aufheizens der Glaspartikel um ein vorgegebenes Maß gegen die Pressrichtung, das insbesondere weniger als 1 mm und besonders bevorzugt zwischen 0,3 und 0,5 mm beträgt.

Für die Erfassung des Mikrobewegung, die sich im Grunde als Mikroimpuls eines Bewegungsparameters darstellt, ist primär ein Wegsensor oder ein Geschwindigkeitssensor geeignet. Die Auflösung sollte mindestens 0,01 mm oder eine entsprechende Geschwindigkeitsgröße betragen, bevorzugt 50 $\mu$m pro Sekunde.

Andernfalls, also beispielsweise, wenn die Presskraft über einen Schrittmotor mit einem nicht elastischen oder gering elastischen Antriebsstrang ausgeübt wird, ist auch die Realisierung eines Kraftsensors oder Drucksensors als Sensor für die Erfassung der Mikroimpulse möglich. Alternativ ist auch eine reine Wegmessung, gegebenenfalls bei einer Presskraft nahe Null, möglich.

Die Amplitude und Häufigkeit der Mikroimpulse hängt selbstverständlich von physikalischen Parametern ab, beispielsweise von dem Material der zu pressenden Glaspartikel, der verwendeten Einbettmasse, der Temperatur, aber auch beispielsweise der aufgebrachten Presskraft bzw. deren Regelung.

Die der Presskraft entgegenwirkende Wärmeausdehnung setzt sich im wesentlichen zusammen aus der Wärmeausdehnung des Pressstempels, des Presskolbens, der Glaspartikel, und der Muffel unterhalb der Glaspartikel. Auch der Isolationsbereich des Ofens unterhalb des Brennraumbodens wird noch etwas miterwärmt und dehnt sich dementsprechend aus.

Besonders günstig läßt sich auch die Tatsache ausnutzen, dass der Pressstempel in Kontakt mit dem zwischengeschalteten Presskolben und somit auch mit dem oberen Ende des Haufwerks der Glaspartikel steht, bevorzugt unter einem Anpressdruck. Die Presskraft kann ohne Zeitverzögerung übertragen werden, ebenso wie die Rückwärtsbewegung bei der Materialausdehnung der Glaspartikel. Die Steuervorrichtung für die Steuerung des Pressvorgangs erfaßt nicht nur diese, sondern steuert auch den weiteren Verlauf des Temperatur-Zeitprofils.

Aufgrund der starken Wärmedämmung ist der Ofen typischerweise außen und damit an den tragenden Stellen, die für das Schließen des Ofens zuständig sind, - zumindest in Relation zu heißen Brennraum betrachtet - praktisch kalt, typischerweise zwischen Raumtemperatur und 50°C, so dass die dort bestehende Erwärmung relativ gering ist.

Hierbei wird davon ausgegangen, dass die Heizung in an sich bekannter Weise als Ringheizung den zylindrischen Brennraum seitlich umschließt und durch Konvektion und Wärmestrahlung hauptsächlich die Muffel, aber auch natürlich das darin befindlichen Haufwerk der Glaspartikel und gegebenenfalls den Presskolben erwärmt, typischerweise in wenigen Minuten um mehrere 100°C. Bei typischen Pressöfen beträgt die Wandstärke der Wärmedämmung fünf Zentimeter oder weniger und liegt damit im Bereich des Durchmessers der Muffel oder darunter.

Die Erwärmung in dem Antriebsstrang aus Presskolben, Pressstempel, Glaspartikeln und Einbettmasse im unteren Teil sowie gegebenenfalls dem Brennteller und umgebenenden Teilen erzeugt einen Gegendruck auf den Pressantrieb.

Erfindungsgemäß ist es möglich, während der erfindungsgemäßen Erfassung des Auslösekriteriums mit der gleichen Presskraft wie während des eigentlichen Pressens zu arbeiten.

Bevorzugt ist es jedoch, eine Hilfspresskraft zwischen dreißig und fünfzig Prozent der Nennpresskraft zu wählen, da diese bereits ausreicht, die erwünschte Bewegungshysterese zu erzeugen.

Es ist auch möglich, die Presskraft unmittelbar nach dem Zustellen des Pressstempels zum Presskolben - und damit indirekt zum Haufwerk der Glaspartikel - kurzzeitig auf einem vergleichweise hohem Wert zu halten, um eine stabile Anlage des Haufwerks der Glaspartikel in der Muffel zu gewährleisten und dann eine Presskraftnachregelung vorzunehmen.

Ein weiterer Gesichtspunkt, der für die Genauigkeit der durchgeführten Messungen relevant ist, ist das Bereitstellen des Ofenunterdrucks vor Vornahme der Messung. Durch den Ofenunterdruck werden die Dichtungen zwischen Ofenhaube und Ofenunterseite, oder beispielsweise zwischen Ofenhaube und einem heranfahrbarem Brenntisch, auf das vorgegebene Maß komprimiert. Die Kompression kann durchaus im Bereich mehrerer Millimeter liegen und würde das Messergebnis verfälschen, wenn der Unterdruck während der Vorpresszeit, also während der Erfassung des Auslösekriteriums, aufgebaut würde.

Wenn - aus welchen Gründen auch immer - während dieser Zeit noch kein vollständiger Unterdruck, wie er während des Presszyklus erwünscht ist, bereitgestellt werden soll, muss zumindest dafür Sorge getragen werden, dass der Unterdruck im Ofeninnenraum während dieser Vorpresszeit konstant bleibt.

Typischerweise lässt sich aber - ausreichende Dichtheit des Ofens vorausgesetzt - der Unterdruck innerhalb einer deutlich geringeren als der erforderlichen Aufheizzeit des Ofens bereitsstellen, beispielsweise innerhalb von 1 bis 2 Minuten.

In einer weiteren bevorzugten Ausführungsform ist es vorgesehen, dass beim Pressvorgang, falls ein vobestimmter Teil des Haufwerks der Glaspartikel erwärmt wird und verdichtet ist, der Unterdruck durch z.B. eine ganze oder teilweise Belüftung der Brennkammer reduziert werden kann. Wenn anschließend ein anderer Bereich der Glaspartikel ebenfalls verdichtet wird, wird dort in den Lücken zwischen den Glaspartikeln dieses Bereichs Luft eingeschlossen und damit eine vollständige Verdichtung dieses Bereichs verhindert. Dadurch ergibt sich z.B. ein Block mit unterschiedlichen Dichten.

Gemäß einer weiteren bevorzugten Ausführungsform ist für den Pressantrieb eine Kraftregelung vorgesehen. Aufgrund des Aufheizens und damit während der Wärmeausdehnung des Antriebsstrangs (einschließlich des Haufwerks der Glaspartikel) muss eine Nachregelung erfolgen, z. B. bei Überschreiten einer vorgegebenen Kraft. Bei entsprechend guter Einstellung dieser Nachregelcharakteristik kann eine qualitativ und quantitativ gut interpretierbare Anzahl von Mikroimpulsen realisiert werden. Während der Erwärmung der Glaspartikel im noch festen Aggregatzustand nimmt der Abstand zwischen den Mikroimpulsen und/oder der Abstand zwischen Intervallen, bei denen eine Nachregelung der Kraft erfolgt, exponentiell zu.

Als Auslösekriterium lässt sich nun beispielsweise ein Ende der Mikroimpulsserie verwenden, angenähert durch das Ausbleiben eines Impulses beispielsweise über mehr als 30 Sekunden.

Es ist aber möglich, kurzerhand die Anzahl der Impulse zu zählen und basierend hierauf das Auslösekriterium festzulegen. Ferner ist es auch möglich, das Maß der Zeitdifferenz zwischen zwei aufeinander folgenden Impulsen als Auslösekriterium zu verwenden.

In einer weiteren bevorzugten Ausführungsform ist es vorgesehen, dass nach dem Pressvorgang ein Kristallisationsprogramm gestartet wird, um gesteuerte, homogene Glaskristalle zu bilden, aber nicht das ungewünschte Kristallwachstum wie bei dem Verdichtungsprozess. Hieraus ergibt sich beispielsweise ein Block aus Lithiummetasilikat.

Bevorzugt übt bei dem Kristallisationsprogramm der Pressstempel keinen Druck mehr aus. Ein zu langes Pressen könnte zu einer unerwünschten oder inhomogenen Kristallisation führen.

Das Kristallisationsprogramm wird bevorzugt in einem Temperaturbereich zwischen dem Transformationsbereich Tg und etwa 80 °C über den Transformationsbereich Tg der Glaspartikel (Tg + 80 °C), insbesondere zwischen 40 °C über den Transformationsbereich Tg (Tg + 40 °C) und etwa 60 °C über den Transformationsbereich Tg der Glaspartikel (Tg + 60 °C), vorgenommen. Bei diesem Temperaturbereich findet eine Keimbildung des Haufwerks von Glaspartikeln statt. Eine darauffolgende Temperaturerhöhung auf 60 °C bis 160 °C über Tg bewirkt eine kontrollierte Kristallisation. Eine noch höhere Temperatur zur Kristallisation ist möglich, würde aber die Pressform unnötig verschleißen.

In einer weiteren bevorzugten Ausführungsform ist es vorgesehen, dass nach dem Pressvorgang und/oder nach dem Kristallisationsprogramm ein Abkühlprozess und/oder ein Entformungsprozess gestartet wird bzw. werden. Bevorzugt wird im Falle einer zu herstellenden dentalen Restauration eine gipsbasierte dentale Einbettmasse in einer Muffel verwendet, welche Einbettmasse gegenüber hochtemperaturbeständigen phosphatgebundenen Einbettmassen zwei Vorteile aufweist. Zum einen ist die Oberflächenqualität und damit die Abbildegenauigkeit besser. Zum anderen ist das Ausbetten leichter.

Im Falle, dass ein Ingot oder ein Fräsblock direkt nach dem Pressvorgang abgekühlt und entformt wird, kann dieser auf etwaige Fehler kontrolliert werden, weil dieser dann glasig tranparent ist.

In einer weiteren bevorzugten Ausführungsform ist es vorgesehen, dass am Ende aller Verfahren sich ein Block aus Lithiumsilikat mit Keimbildnern ergibt.

Zum Ofen der vorliegenden Erfindung gehört jede beliebige Heizvorrichtung, die Wärmeenergie bereitstellt.

Die Glaspartikel der vorliegenden Erfindung beziehen sich hauptsächlich auf Glaspulver, Glasgranulat und Glassand, aber beschränkt sich nicht hierauf. Alle industriell anwendbaren Glaspartikel, insbesondere diejenigen für die Herstellung von Dentalrestaurationsteilen, liegen im Schutzbereich der vorliegenden Erfindung.

Weitere Vorteile, Einzelheiten und Merkmale ergeben sich aus der nachfolgenden Beschreibung eines Ausführungsbeispiels der Erfindung anhand der Zeichnungen.

Es zeigen:

Fig. 1 einen Schnitt durch den Brennraum eines erfindungsgemäßen Ofens in einer Ausführungsform;

Fig. 2 ein Diagramm der Geschwindigkeit des Pressstempels und weiterer physikalischer Größen, aufgetragen über die Zeit; und

Fig. 3 ein schematisch dargestelltes Diagramm der Geschwindigkeit der Keimbildung, der Verdichtung und des Kristallwachstums, aufgetragen über die Temperatur.

In Fig. 1 ist ein erfindungsgemäßer Ofen 10, der insbesondere für die Herstellung von Dentalrestaurationsteilen geeignet ist, in einem für die Erfindung relevanten Ausschnitt dargestellt.

Ein Brennraum 12 ist von einer schematisch dargestellten Heizvorrichtung 14 mit einer spiralig verlaufenden Heizwendel umgeben, die von einem Quarzglas 16 als Schutzvorrichtung abgeschirmt ist. Großvolumige Wärmedämmelemente 18 umgeben den Brennraum 12 allseitig, also auch nach unten, auch wenn dies aus Fig. 1 nicht ersichtlich ist.

Der Boden des Brennraums 12 ist von einem Brennteller 20 gebildet, der Vertiefungen 22 für die Aufnahme der Muffel 24 aufweist, und zwar abgestuft in unterschiedlichen Größen, in dem dargestellten Ausführungsbeispiel in zwei Größen.

Der Brennraum 12 weist einen Dachkonus 26 auf, der den Brennraum nach oben hin zentral vergrößert. In diesem Bereich ist ein Teil eines Presskolbens 28 aufgenommen, der in einem Presskanal 30 eingesteckt ist, anliegend an ein Haufwerk von Glaspartikeln 32 aus Lithiumsilikat, das vollständig in der Muffel 24 in dem Presskanal 30 aufgenommen ist und in der dargestellten Ausführungsform Farb- und Transluzenzgradienten bildet.

Der Presskolben kann aus einem beliebigen geeigneten Material bestehen, beispielsweise aus $Al_2O_3$, Bornitrid, Grafit und/oder aus Einbettmasse selbst.

In einem Pressstempelkanal ist ein Pressstempel 36 geführt, der über eine Antriebsvorrichtung angetrieben ist und Druck auf den Presskolben 28 und damit indirekt auf das Haufwerk von Glaspartikeln 32 auszuüben vermag.

Für den Betrieb des Ofens wird dieser nun zunächst geöffnet. Eine in einem sogenannten Vorwärmofen beispielsweise auf 850°C vorgewärmte Muffel, in die ein kaltes Haufwerk von Glaspartikeln 32 und ein kalter Presskolben 28 bereits eingeführt sind, wird auf den Brennteller 20 zentral abgestellt. Die Vertiefung 22 passt in ihren Abmessungen exakt zu der zugehörigen Muffel 24, so dass die Muffel 24 exakt zentral steht. Die Ofenhaube wird geschlossen und eine Unterdruckquelle saugt die in dem Brennraum 12 und in den Wärmedämmelementen vorhandene Luft ab, bis ein Unterdruck entsteht.

Dichtungen sind zwischen einem Ofenunterbau und der Brennhaube des Ofens vorgesehen, die durch den Aufbau des Unterdrucks zusammengedrückt werden. Der Aufbau des Unterdrucks ist in ein bis zwei Minuten erfolgt, und während des gesamten folgenden Presszyklus wird der Unterdruck konstant gehalten, beispielsweise durch eine Druckregelung der Unterdruckquelle, oder aber, indem die entsprechende Saugpumpe laufen gelassen wird.

Sobald das Haufwerk von Glaspartikeln 32 in die Muffel 24 eingeführt ist, beginnt die Erwärmung der Glaspartikel 32, die gegenüber der Muffel 24 eine wesentlich geringere Masse aufweisen. Das Haufwerk der Glaspartikel dehnt sich hierdurch aus, wobei die Vorwärmtemperatur deutlich unterhalb der Erweichungstemperatur liegt. Der Wärmeausdehnungskoeffizient der Muffel 24, also der Einbettmasse, die für die Ausbildung der Muffel verwendet wird, ist deutlich geringer als der Wärmeausdehnungskoeffizient des Haufwerks der Glaspartikel, aber auch des Presskolbens und des Pressstempels, wobei der Pressstempel beispielsweise ebenfalls aus $Al_2O_3$ bestehen kann oder aber beispielsweise auch aus Stahl.

Im hier dargestellten Ausführungsbeispiel beträgt der Wärmeausdehnungskoeffizient der Muffel 3 x 10-6/K und der der jeweiligen Glaspartikel etwa 10 x 10-6/K, und derjenige des Pressstempels 8 x 10-6/K.

Bei der hier betrachteten Wärmeausdehnung in axialer Richtung addieren sich die Wärmeausdehnungen $L_0$ bei Erwärmung. Die Gesamtwärmeausdehnung $L_{0\ ges}$ beträgt:

$$L_{0\ ges} = L_{0\ EBM} + L_{0\ Haufwerk} + L_{0\ Aloxkolben} + L_{0\ Pressstempel}$$

Hierbei muss der Pressstempel 36 nur soweit berücksichtigt werden, wie er erwärmt wird, also in dem dem Brennraum benachbarten Bereich der Wärmedämmelemente 18.

Das gegenüberliegende Ende des Presstempels 36, das mit der Antriebsvorrichtung verbunden ist, ist deutlich weniger heiß, beispielsweise unter 100°C.

Der Temperaturgradient des Pressstempels 36 ist insbesondere dann hoch, wenn ein Pressstempel beispielsweise aus $Al_2O_3$ verwendet wird; bei Verwendung eines metallischen Pressstempels kann auch ein zusätzlicher Wärmedämmzylinder in den Pressstempel eingebaut sein.

$L_{0\ EBM}$ bedeutet die axiale Länge der Muffel oder Einbettmasse unterhalb des Presskanals 30.

Bei der betrachteten Ausführungsform des Ofens ist der Brennteller 20 lediglich oberflächlich warm und im unteren Bereich bereits vergleichsweise kühl. Dies gilt jedoch nicht, wenn eine sogenannte Fußheizung eines Brennraums realisiert ist, also wenn unterhalb des Brenntellers 22 eine weitere Heizung vorgesehen ist. Bei dieser Ausführungsform muss die zusätzliche Wärmeausdehnung im dortigen Bereich zu der vorstehenden Gesamtwärmeausdehnung hinzuaddiert werden.

Um den axialen Versatz des Pressstempels 36 durch die Wärmeausdehnung zu ermitteln, ist von $L_{0\ ges}$ die Wärmeausdehnung des Ofens selbst bei der Erwärmung abzuziehen. Hierbei ist jedoch zu berücksichtigen, dass typischerweise die Dichtung und die Schließkraft des Ofens weit außen realisiert sind und dass dort die Wärmeausdehnung typischerweise auf den Bereich zwischen Raumtemperatur und einer Temperatur unter 100°C, beispielsweise 60°C, beschränkt bleibt; die Wärmedämmelemente 18, die zumindest innen sehr heiß werden, üben keine axiale Kraft aus, sondern liegen locker in dem Ofen auf.

Unter axial sei hier die Achse 40 des Pressstempels 36 zu verstehen, die zentral sowohl durch den Presskolben als auch das Haufwerk der Glaspartikel 32 als auch die Muffel 24 als auch den Brennteller 20 hindurch verläuft; sämtliche Teile sind in dem dargestellten Ausführungsbeispiel kreissymmetrisch ausgebildet.

In Fig. 2 ist eine Vielzahl von Kurven im gleichen Diagramm eingetragen, wobei die Abszisse die Zeit ist. Hierbei handelt es sich unter anderem um folgende Werte:

Die Temperaturkurve 51 zeigt die geregelte Temperatur im Brennraum des Ofens.

Die Druckkurve 52 zeigt den Druck im Innenraum des Ofens.

Die Geschwindigkeitskurve 53 zeigt die Geschwindigkeit des Pressstempels 36 beziehungsweise der zugehörigen Antriebsvorrichtung.

Der Nullpunkt der Ordinate ist hierbei höhenversetzt und die Geschwindigkeitswerte in μm/min sind rechts eingetragen.

Die aufgebrachte Presskraft ist in der Presskraftkurve 54 eingetragen.

Wie aus Fig. 2 ersichtlich ist, ist der Ofen 10 beim betrachteten Beginn bereits vorgeheizt, auf etwa 550°C. Die Heizung ist weiter eingeschaltet. Zu dem Zeitpunkt etwa 70 Sekunden ab Beginn wird die Aufheizung vorgenommen, wobei die Zieltemperatur im betrachteten Ausführungsbeispiel bei etwa 580 °C liegt. Nach 10 Sekunden bei etwa 80 Sekunden wird mit dem dortigen Peak der Geschwindigkeit der Pressstempel 36 rasch in den Presskanal 30 eingeführt, bis Pressstempel, Presskolben und das Haufwerk der Glaspartikel aufeinander aufliegen. Der Pressstempel 36 übt auf das Haufwerk der Glaspartikel 32 einen konstanten Druck von ca. 100 N aus.

Wie aus der Druckkurve 52 ersichtlich ist, nimmt der Druck im Ofen innerhalb der ersten 40 Sekunden rasch ab und beträgt bei 70 Sekunden 100 mbar. Spätestens bei 240 Sekunden ist der Enddruck von 50 mbar erreicht. Dieser wird während des gesamten Presszyklus konstant gehalten.

Von 70 Sekunden bis ca. 115 Sekunden beträgt die Geschwindigkeit des Pressstempels 36 null, wie aus Fig. 2 ersichtlich ist. Bei etwas über 115 Sekunden entsteht ein erfindungsgemäßer Mikroimpuls 60 mit einer negativen Geschwindigkeit. Dieser entspricht einer Wärmeausdehnung $L_{0\ ges}$, wie es mit Bezug auf Fig: 1 erläutert wurde. Der Mikroimpuls hat eine Größe von etwa 500 μm/min und eine Dauer von weniger als einer Sekunde, abhängig von der Güte der Presskraftregelung - wenn dies auch aus Fig. 2 basierend auf der hier dargestellten Ausführung nicht klar ersichtlich ist.

Das obere Ende des Haufwerks der Glaspartikel 32 rutscht gleichsam ein bisschen nach oben, entgegen der Wirkung des Pressdrucks gemäß der Presskurve 54, und fällt in kurzer Zeit aufgrund der Erweichung der Glaspartikel wieder ab. Zu dem Zeitpunkt etwa 135 Sekunden erfolgt der nächste Mikroimpuls 62. Hieran schließen sich weitere Mikroimpulse 64, 66, 68 und 70 bei 150, 170, 195 und 225 Sekunden an, während der folgende Zeitabschnitt bis 250 Sekunden mikroimpulsfrei verbleibt.

In dem dargestellten Ausführungsbeispiel wird erfindungsgemäß durch die Erfassung einer erfahrungsgemäß vorgegebenen Anzahl der Mikroimpulse der Pressprozess ausgelöst, wenn die Anzahl der Mikroimpulse gemäß Fig. 2 zu sechs erreicht.

Dies bedeutet erfindungsgemäß, dass die Glaspartikel 32 mikroplastisch beziehungsweise viskos geworden sind; die weitere Wärmeausdehnung erfolgt impulsfrei. Diese Tatsache wird erfindungsgemäß als Indiz gewertet, dass das Haufwerk der Glaspartikel die tatsächlich erwünschte Temperatur zur Vorbereitung des Pressvorgangs erreicht hat.

Für die Erfassung der Mikroimpulse ist es grundsätzlich möglich, eine Geschwindigkeitserfassung, eine Wegerfassung oder aber eine Presskrafterfassung vorzunehmen, je nach Auflösung der zur Verfügung stehenden Sensoren und je nach Elastizität des Antriebs. Bei Versuchen im Zusammenhang mit dem hier zu Rede stehendem Ausführungsbeispiel hat sich eine Geschwindigkeitserfassung als die vorteilhafteste herausgestellt, wobei allerdings auch eine Wegerfassung grundsätzlich möglich ist.

Obwohl aus Fig. 2 die gesamte Aufheizzeitdauer des Haufwerks der Glaspartikel bis zum Beginn der Verdichtung nicht ersichtlich ist, ist hier zu bemerken, dass erfindungsgemäß eine gegenüber dem Stand der Technik beträchtlich schnellere Aufheizung vorgenommen wird. Dadurch wird eine kontrollierte, geringe Menge von Keimen erzeugt.

Vom Zeitpunkt 250 Sekunden an beginnt der Verdichtungsprozess bis etwa 660 Sekunden. Die Verdichtung erreicht ihre maximale Geschwindigkeit inzwischen beim Zeitpunkt ca. 470 Sekunden.

In einer bevorzugten Ausführungsform ist es vorgesehen, dass sofort nach dem Verdichtungsprozess ein Abkühlprogramm gestartet wird, um das ansonsten bei höherer Temperatur erfolgende Kristallwachstum zu verhindern.

In einer weiteren bevorzugten Ausführungsform ist es vorgesehen, dass nach dem Verdichtungsprozess ein Kristallisationsprogramm gestartet wird, so dass sich gewünschtes, homogenes und feineres Kristallgefüge ergibt.

In einer weiteren bevorzugten Ausführungsform ist es vorgesehen, dass nach dem Verdichtungsprozess ein Abkühlprogramm und/oder ein Kristallisationsprogramm und/oder ein Entformungsprogramm und/oder ein Reinigungsprogramm vorgenommen wird. Dadurch ergibt sich als Beispiel ein Block aus Lithiummetasilikat-Glaskeramik oder aus Lithiumsilikatglas mit Keimbildnern.

In Fig. 3 sind drei Kurven 74, 74, 76 in einem Diagramm eingetragen, wobei die Abszisse die Temperatur ist. Die Geschwindigkeitskurven 72, 74, 76 je mit einer Vielzahl von Dreiecken, Kreisen und Quadraten zum Unterscheiden voneinander zeigen unter unterschiedlichen Temperaturen die Geschwindigkeit der Keimbildung, der Verdichtung und des Kristallwachtums der Glaspartikel aus Lithiumsilikat.

Der Nullpunkt der Abszisse - also der Temperatur - ist hierbei höhenversetzt und entspricht dem Transformationsbereich Tg von Gläsern aus Lithiumsilikat, der gegen 450 °C liegt. Bei dem Transformationsbereich Tg beträgt die Viskosität η der Glaspartikel aus Lithiumsilikat etwa 10exp13,2 Poise und haben sich die Mikroimpulse bereit geendet, solange die thermische Ausdehnung des Haufwerks der Glaspartikel durch mikroplastische Verformung an den Kontaktstellen der Glaspartikel kompensiert wird und die Viskosität η eine Höhe von 10exp14,5 Poise erreicht.

Aus Fig. 3 ist ersichtlich, dass eine Keimbildung von Gläsern aus Lithiumsilikat im Temperaturbereich zwischen 460 bis 530 °C, also zwischen 10 °C über den Transformationsbereich Tg der Glaspartikel (Tg + 10 °C) und 80 °C über den Transformationsbereich Tg der Glaspartikel (Tg + 80 °C), eine Verdichtung dieser im Temperaturbereich zwischen 500 bis 580 °C, also zwischen 50 °C über den Transformationsbereich Tg der Glaspartikel (Tg + 50 °C) und 130 °C über den Transformationsbereich Tg der Glaspartikel (Tg + 130 °C), ein Kristallwachstum dieser im Temperaturbereich zwischen 510 bis 610 °C, also zwischen ca. 60 °C über den Transformationsbereich Tg der Glaspartikel (Tg + 60 °C) und 160 °C über den Transformationsbereich Tg der Glaspartikel (Tg + 160 °C) stattfindet.

Diese drei Temperaturbereiche überlappen sich, und in dieser Temperaturbereiche befinden sich je eine maximale Geschwindigkeit der Keimbildung (bei ca. 500 °C), eine maximale Geschwindigkeit der Verdichtung (bei ca. 550 °C) und eine maximale Geschwindigkeit des Kristallwachtums (bei ca. 580 °C).

Um vor und bei der Verdichtung lediglich wenige Keime (in gewünschtem Maße) zu bilden, damit eine vollständige Verdichtung nicht durch die Keime stark verhindert wird, wird erfindungsgemäß eine schnelle Aufheizung, beispielsweise innerhalb drei Minuten, von 460 °C (Tg + 10 °C) auf 520 °C (Tg + 70 °C) entsprechend dem Temperaturpunkt 78 gemäß Fig. 3 vorgenommen.

Bei dem Temperaturpunkt 78 weisen sowohl die Keimbildung, als auch das Kristallwachstum eine relativ kleine Geschwindigkeit auf, während die Geschwindigkeit der Verdichtung relativ groß ist. Erfindungsgemäß wird daher dieser Temperaturpunkt 87 ausgenutzt. Durch eine schnelle Aufheizung auf den Temperaturpunkt 87 werden vor und bei der Verdichtung möglichst wenige Keime und Glaskristalle gebildet.

Außer dem optimalen Temperaturpunkt 78 kann die Ende-Temperatur für die schnelle Aufheizung eine andere vorgegebene Temperatur zwischen 500 °C (Tg + 50 °C) und 530 °C (Tg + 80 °C) sein, welche mindestens der dilatometrischen Erweichung der Glaspartikel entspricht und bei welcher die Viskosität der Glaspartikel $\eta$ mindestens 10exp11,5 Poise beträgt.

**Patentansprüche**

1. Anordnung eines Ofens und eines Haufwerks von Glaspartikeln mit einem Haufwerk von Glaspartikeln (32) und einem Ofen (10), welcher einen Pressstempel (36), einen Druck-, Weg- und/oder Geschwindigkeits-Sensor und eine Steuervorrichtung für die Steuerung eines Pressvorgangs basierend auf dem Ausgangssignal des Sensors aufweist, wobei der Sensor mindestens einen Druck-, Orts-, und/oder Bewegungsparameter des Pressstempels (36) erfasst, wobei der Pressstempel (36) - gegebenenfalls über einen zwischengeschalteten Presskolben (28) - auf das Haufwerk von Glaspartikeln (32) wirkt, die in einem Presskanal (30) geführt und insbesondere kristallisierbar sind, und wobei das Auslösekriterium für die Prozesssteuerung eine über den Sensor erfasste Änderung mindestens eines Bewegungsparameters des Pressstempels (36) bei Erweichung des Haufwerks von Glaspartikeln (32) ist.

2. Anordnung eines Ofens und eines Haufwerks von Glaspartikeln nach Anspruch 1, **dadurch gekennzeichnet, dass** die Prozesssteuerung den Start des Pressvorgangs steuert, bei welchem Start die Ofentemperatur, der Druck in einem Brennraum (12) des Ofens (10) und eine Presskraft des Pressstempels (36) durch die Steuervorrichtung gesteuert werden, und dass die Prozesssteuerung das Ende des Pressvorgangs steuert, bei welchem Ende die Presskraft des Pressstempels (36) durch die Steuervorrichtung, insbesondere auf Null, reduziert und die Ofentemperatur durch die Steuervorrichtung, insbesondere mittels der Abschaltung des Ofens (10), reduziert wird.

3. Anordnung eines Ofens und eines Haufwerks von Glaspartikeln nach Anspruch 1, **dadurch gekennzeichnet,**

   - **dass** das Haufwerk von Glaspartikeln (32) im Presskanal (30) Gradienten, insbesondere Farb- und/oder Transluzenzgradienten, bildet, und/oder
   - **dass** die Gradienten mindestens einer graduellen schichtweisen und/oder einer kontinuierlichen Änderung einer physikalischen Eigenschaft des Haufwerks von Glaspartikeln entsprechen, und/oder
   - **dass** das Haufwerk von Glaspartikeln (32) aus verschiedenen eingefärbten Glaspartikeln und/oder mindestens einer Glassorte, die mit Pigmenten unterschiedlich eingefärbt wird, und/oder aus Gläsern mit unterschiedlichen Gehalten an Keimbildnern und/oder mit unterschiedlichen Dichten besteht.

4. Anordnung eines Ofens und eines Haufwerks von Glaspartikeln nach einem der vorhergehenden Ansprüche, **dadurch gekennzeichnet,**

   - **dass** das Auslösekriterium für den Start des Pressvorgangs die Beendigung von Mikroimpulsen (60, 62, 64, 66, 68, 70) des Bewegungsparameters und/oder deren Anzahl und/oder deren zeitlicher Abstand ist, und/oder
   - **dass** die Mikroimpulse (60, 62, 64, 66, 68, 70) eine kurzzeitige und geringe Bewegung zwischen dem Haufwerk von Glaspartikeln (32) und der Muffel (24) wiedergeben, und/oder
   - **dass** die Steuervorrichtung die Anzahl der Mikroimpulse (60, 62, 64, 66, 68, 70) der Bewegungsparameter und/oder deren Abstand und/oder deren Änderung des Abstands und/oder deren Größe erfasst und für die Prozesssteuerung verwendet.

**5.** Anordnung eines Ofens und eines Haufwerks von Glaspartikeln nach einem der vorhergehenden Ansprüche, **dadurch gekennzeichnet, dass** das Auslösekriterium für das Ende des Pressvorgangs der Rückgang der Pressgeschwindigkeit auf einen Wert nahe Null oder einen Wert von weniger als 5 %, insbesondere weniger als 2 %, der maximalen Pressgeschwindigkeit des Pressstempels (36) ist.

**6.** Anordnung eines Ofens und eines Haufwerks von Glaspartikeln nach einem der vorhergehenden Ansprüche, **dadurch gekennzeichnet, dass** die Steuervorrichtung eine Rückwärtsbewegung des Pressstempels (36) entsprechend der Wärmeausdehnung des Haufwerks von Glaspartikeln (32) über den Sensor erfasst und das Auslösekriterium für den Start des Pressvorgangs ein Ende der Rückwärtsbewegung und/oder der Beginn einer Vorwärtsbewegung und/oder ein definierter Zeitpunkt nach einer Änderung von Bewegungsparametern des Pressstempels (36) ist.

**7.** Anordnung eines Ofens und eines Haufwerks von Glaspartikeln nach einem der vorhergehenden Ansprüche, **dadurch gekennzeichnet, dass** der Sensor unterhalb der Erweichungstemperatur des Haufwerks von Glaspartikeln (32) die der Presskraft entgegenwirkende Wärmeausdehnung der Muffel (24), gegebenenfalls des Presskolbens (28), des Pressstempels (36) und des Haufwerks von Glaspartikeln (32), insbesondere die aufsummierte Wärmeausdehnung dieser, erfasst.

**8.** Anordnung eines Ofens und eines Haufwerks von Glaspartikeln nach einem der vorhergehenden Ansprüche, **dadurch gekennzeichnet,**

- **dass** der Sensor die der Erweichung des Haufwerks von Glaspartikeln (32) entsprechende abnehmende Gegenkraft aufgrund von Wärmeausdehnungskoeffizienten oder aufgrund von Differenzen zwischen Wärmeausdehnungskoeffizienten der Steuervorrichtung zuleitet, wobei diese einen weiteren Prozessverlauf auf einen Zeitpunkt nach dem Zeitpunkt der Erfassung der Erweichung des Haufwerks von Glaspartikeln (32) festlegt, und/oder
- **dass** die Steuervorrichtung den Pressstempel (36) vor den eigentlichen Pressvorgang - gegebenenfalls über den zwischengeschalteten Presskolben (28) - an das Haufwerk von Glaspartikeln (32) anlegt und die Wärmeausdehnung des Haufwerks von Glaspartikeln (32) - entsprechend einer Bewegung des Pressstempels (36) gegen die Pressrichtung - erfasst und beim Ausbleiben der weiteren Wärmeausdehnung beziehungsweise bei der Erweichung die abnehmende Gegenkraft als insbesondere zusätzliches Auslösekriterium heranzieht, und/oder
- **dass** die Steuervorrichtung den Pressstempel (36) eine Presskraft beziehungsweise einen Pressdruck auf das Haufwerk von Glaspartikeln (32) ausüben lässt, auch bevor das Haufwerk von Glaspartikeln (32) erweicht ist.

**9.** Anordnung eines Ofens und eines Haufwerks von Glaspartikeln nach einem der vorhergehenden Ansprüche, **dadurch gekennzeichnet, dass** basierend auf dem von dem Sensor erfassten Bewegungsparameter, dem von dem Pressstempel (36) ausgeübten Pressdruck und/oder der Temperatur eines Temperatursensors, der im oder am Brennraum (12) angebracht ist, die Steuervorrichtung die Art und das Material des Haufwerks von Glaspartikeln (32) - basierend auf vorgegebenen Signaturen - erkennt und basierend hierauf den Pressvorgang steuert.

**10.** Anordnung eines Ofens und eines Haufwerks von Glaspartikeln nach einem der vorhergehenden Ansprüche, **dadurch gekennzeichnet, dass** während der Erfassung des Bewegungsparameters der Pressstempel (36) eine konstante Kraft oder eine einem vorgegebenen Profil folgende Kraft auf das Haufwerk von Glaspartikeln (32) - gegebenenfalls über den zwischengeschalteten Presskolben (28) - ausübt und der Bewegungsparameter erfasst wird, nachdem der Pressstempel (36) - gegebenenfalls über den Presskolben (28) - an dem Haufwerk von Glaspartikeln (32) anliegt.

**11.** Anordnung eines Ofens und eines Haufwerks von Glaspartikeln nach einem der vorhergehenden Ansprüche, **dadurch gekennzeichnet, dass** der Pressstempel (36) sich während des Aufheizens des Haufwerks von Glaspartikeln (32) um ein vorgegebenes Maß bewegt, das insbesondere weniger als 1 mm und besonders bevorzugt zwischen 0,3 und 0,5 mm beträgt.

**12.** Verfahren zum Betrieb eines Ofens basierend auf einer Steuervorrichtung für die Steuerung eines Pressvorgangs, wobei ein Druck-, Weg- und/oder Geschwindigkeitssensor mindestens einen Druck-, Orts-, und/oder Bewegungsparameter eines Pressstempels (36) erfasst, und wobei die Steuervorrichtung den Pressvorgang basierend auf einem Ausgangssignal des Sensors steuert, wobei beim Pressvorgang der Pressstempel (36) - gegebenenfalls über einen zwischengeschalteten Presskolben (28) - auf ein Haufwerk von Glaspartikeln (32) wirkt und Druck ausübt, welches Haufwerk von Glaspartikeln (32) in einen Presskanal (30) einer Muffel (24) eingeführt ist, und wobei das Auslösekriterium für die Prozesssteuerung eine über den Sensor erfasste Änderung mindestens eines Bewegungsparameters des Pressstempels (36), entsprechend der Erweichung des Haufwerks von Glaspartikeln (32), ist.

**13.** Verfahren nach Anspruch 12, **dadurch gekennzeichnet, dass** nach der Beendigung von Mikroimpulsen (60, 62, 64, 66, 68, 70) des Bewegungsparameters das Haufwerk von Glaspartikeln (32) innerhalb zehn Minuten, insbesondere innerhalb fünf Minuten, bevorzugt innerhalb drei Minuten auf eine vorgegebene Temperatur erhitzt wird, welche mindestens der dilatometrischen Erweichung des Haufwerks von Glaspartikeln entspricht.

**14.** Verfahren nach dem Anspruch 12 oder 13, **dadurch gekennzeichnet,**

- **dass** nach dem Pressvorgang ein Kristallisationsprogramm gestartet wird, bei dem der Pressstempel (36) keinen Druck mehr ausübt, und/oder
- **dass** im Temperaturenbereich zwischen dem Transformationsbereich bzw. Transformationspunkt Tg des Haufwerks von Glaspartikeln und 100 °C über den Transformationsbereich Tg (Tg + 100 °C), insbesondere zwischen 30 °C über den Transformationsbereich Tg (Tg + 30 °C) und 80 °C über den Transformationsbereich Tg (Tg + 80 °C), eine Keimbildung des Haufwerks von Glaspartikeln (32) stattfindet, und/oder
- **dass** nach dem Pressvorgang und/oder nach dem Kristallisationsprogramm ein Abkühlprozess und/oder ein Entformungsprozess gestartet wird bzw. werden, wobei insbesondere eine gipsbasierte dentale Einbettmasse in einer Muffel (24) verwendet wird.

**15.** Verfahren nach einem der Ansprüche 12 bis 14, **dadurch gekennzeichnet, dass** am Ende des Verfahrens sich ein Block aus Lithiummetasilikat und/oder aus Lithiumsilikat mit Keimbildnern ergibt.

**Geänderte Patentansprüche gemäss Regel 137(2) EPÜ.**

**1.** Anordnung eines Ofens und eines Haufwerks von Glaspartikeln, welcher Ofen (10) einen Pressstempel (36), einen Druck-, Weg- und/oder Geschwindigkeits-Sensor und eine Steuervorrichtung für die Steuerung eines Pressvorgangs basierend auf dem Ausgangssignal des Sensors aufweist, wobei der Sensor mindestens einen Druck-, Orts-, und/oder Bewegungsparameter des Pressstempels (36) erfasst, wobei der Pressstempel (36) - gegebenenfalls über einen zwischengeschalteten Presskolben (28) - auf das Haufwerk von Glaspartikeln (32) wirkt, die in einem Presskanal (30) geführt und kristallisierbar sind, und wobei das Auslösekriterium für die Prozesssteuerung eine über den Sensor erfasste Änderung mindestens eines Bewegungsparameters des Pressstempels (36) bei Erweichung des Haufwerks von Glaspartikeln (32) ist.

**2.** Anordnung eines Ofens und eines Haufwerks von Glaspartikeln nach Anspruch 1, **dadurch gekennzeichnet, dass** die Prozesssteuerung den Start des Pressvorgangs steuert, bei welchem Start die Ofentemperatur, der Druck in einem Brennraum (12) des Ofens (10) und eine Presskraft des Pressstempels (36) durch die Steuervorrichtung gesteuert werden, und dass die Prozesssteuerung das Ende des Pressvorgangs steuert, bei welchem Ende die Presskraft des Pressstempels (36) durch die Steuervorrichtung, Insbesondere auf Null, reduziert und die Ofentemperatur durch die Steuervorrichtung, insbesondere mittels der Abschaltung des Ofens (10), reduziert wird.

**3.** Anordnung eines Ofens und eines Haufwerks von Glaspartikeln nach Anspruch 1, **dadurch gekennzeichnet,**

- **dass** das Haufwerk von Glaspartikeln (32) im Presskanal (30) Gradienten, Insbesondere Farb- und/oder Transluzenzgradienten, bildet.

**4.** Anordnung eines Ofens und eines Haufwerks von Glaspartikeln nach Anspruch 1 oder 3, **dadurch gekennzeichnet, dass** die Gradienten mindestens einer graduellen schichtweisen und/oder einer kontinuierlichen Änderung einer physikalischen Eigenschaft des Haufwerks von Glaspartikeln entsprechen.

**5.** Anordnung eines Ofens und eines Haufwerks von Glaspartikeln nach einem der Ansprüche 1, 3 und 4, **dadurch gekennzeichnet, dass** das Haufwerk von Glaspartikeln (32) aus verschiedenen eingefärbten Glaspartikeln und/oder mindestens einer Glassorte, die mit Pigmenten unterschiedlich eingefärbt wird, und/oder aus Gläsern mit unterschiedlichen Gehalten an Keimbildnern und/oder mit unterschiedlichen Dichten besteht.

6. Anordnung eines Ofens und eines Haufwerks von Glaspartikeln nach einem der vorhergehenden Ansprüche, **dadurch gekennzeichnet,**

   - **dass** das Auslösekriterium für den Start des Pressvorgangs die Beendigung von Mikroimpulsen (60, 62, 64, 66, 68, 70) des Bewegungsparameters und/oder deren Anzahl und/oder deren zeitlicher Abstand ist.

7. Anordnung eines Ofens und eines Haufwerks von Glaspartikeln nach einem der vorhergehenden Ansprüche, **dadurch gekennzeichnet, dass** die Mikroimpulse (60, 62, 64, 66, 68, 70) eine kurzzeitige und geringe Bewegung zwischen dem Haufwerk von Glaspartikeln (32) und der Muffel (24) wiedergeben.

8. Anordnung eines Ofens und eines Haufwerks von Glaspartikeln nach einem der vorhergehenden Ansprüche, **dadurch gekennzeichnet, dass** die Steuervorrichtung die Anzahl der Mikroimpulse (60, 62, 64, 66, 68, 70) der Bewegungsparameter und/oder deren Abstand und/oder deren Änderung des Abstands und/oder deren Größe erfasst und für die Prozesssteuerung verwendet.

9. Anordnung eines Ofens und eines Haufwerks von Glaspartikeln nach einem der vorhergehenden Ansprüche, **dadurch gekennzeichnet, dass** das Auslösekriterium für das
Ende des Pressvorgangs der Rückgang der Pressgeschwindigkeit auf einen Wert nahe Null oder einen Wert von_weniger als 5 %, insbesondere weniger als 2 %, der maximalen Pressgeschwindigkeit des Pressstempels (36) ist.

10. Anordnung eines Ofens und eines Haufwerks von Glaspartikeln nach einem der vorhergehenden Ansprüche, **dadurch gekennzeichnet, dass** die Steuervorrichtung eine Rückwärtsbewegung des Pressstempels (36) entsprechend der Wärmeausdehnung des Haufwerks von Glaspartikeln (32) über den Sensor erfasst und das Auslösekriterium für den Start des Pressvorgangs ein Ende der Rückwärtsbewegung und/oder der Beginn einer Vorwärtsbewegung und/oder ein definierter Zeitpunkt nach einer Änderung von Bewegungsparametern des Pressstempels (36) ist.

11. Anordnung eines Ofens und eines Haufwerks von Glaspartikeln nach einem der vorhergehenden Ansprüche, **dadurch gekennzeichnet, dass** der Sensor unterhalb der Erweichungstemperatur des Haufwerks von Glaspartikeln (32) die der Presskraft entgegenwirkende Wärmeausdehnung der Muffel (24), gegebenenfalls des Presskolbens (28), des Pressstempels (36) und des Haufwerks von Glaspartikeln (32), insbesondere die aufsummierte Wärmeausdehnung dieser, erfasst.

12. Anordnung eines Ofens und eines Haufwerks von Glaspartikeln nach einem der vorhergehenden Ansprüche, **dadurch gekennzeichnet,**

   - **dass** der Sensor die der Erweichung des Haufwerks von Glaspartikeln (32) entsprechende abnehmende Gegenkraft aufgrund von Wärmeausdehnungskoeffizienten oder aufgrund von Differenzen zwischen Wärmeausdehnungskoeffizienten der Steuervorrichtung zuleitet, wobei diese einen weiteren Prozessverlauf auf einen Zeitpunkt nach dem Zeitpunkt der Erfassung der Erweichung des Haufwerks von Glaspartikeln (32) festlegt.

13. Anordnung eines Ofens und eines Haufwerks von Glaspartikeln nach einem der vorhergehenden Ansprüche, **dadurch gekennzeichnet, dass** die Steuervorrichtung den Pressstempel (36) vor den eigentlichen Pressvorgang - gegebenenfalls über den zwischengeschalteten Presskolben (28) - an das Haufwerk von Glaspartikeln (32) anlegt und die Wärmeausdehnung des Haufwerks von Glaspartikeln (32) - entsprechend einer Bewegung des Pressstempels (36) gegen die Pressrichtung - erfasst und beim Ausbleiben der weiteren Wärmeausdehnung beziehungsweise bei der Erweichung die abnehmende Gegenkraft als insbesondere zusätzliches Auslösekriterium heranzieht.

14. Anordnung eines Ofens und eines Haufwerks von Glaspartikeln nach einem der vorhergehenden Ansprüche, **dadurch gekennzeichnet, dass** die Steuervorrichtung den Pressstempel (36) eine Presskraft beziehungsweise einen Pressdruck auf das Haufwerk von Glaspartikeln (32) ausüben lässt, auch bevor das Haufwerk von Glaspartikeln (32) erweicht ist.

15. Anordnung eines Ofens und eines Haufwerks von Glaspartikeln nach einem der vorhergehenden Ansprüche, **dadurch gekennzeichnet, dass** basierend auf dem von dem Sensor erfassten Bewegungsparameter, dem von dem Pressstempel (36) ausgeübten Pressdruck und/oder der Temperatur eines Temperatursensors, der im oder am Brennraum (12) angebracht ist, die Steuervorrichtung die Art und das Material des Haufwerks von Glaspartikeln (32) - basierend auf vorgegebenen Signaturen - erkennt und basierend hierauf den Pressvorgang steuert.

**16.** Anordnung eines Ofens und eines Haufwerks von Glaspartikeln nach einem der vorhergehenden Ansprüche, **dadurch gekennzeichnet, dass** während der Erfassung des Bewegungsparameters der Pressstempel (36) eine konstante Kraft oder eine einem vorgegebenen Profil folgende Kraft auf das Haufwerk von Glaspartikeln (32) - gegebenenfalls über den zwischengeschalteten Presskolben (28) - ausübt und der Bewegungsparameter erfasst wird, nachdem der Pressstempel (36) - gegebenenfalls über den Presskolben (28) - an dem Haufwerk von Glaspartikeln (32) anliegt.

**17.** Anordnung eines Ofens und eines Haufwerks von Glaspartikeln nach einem der vorhergehenden Ansprüche, **dadurch gekennzeichnet, dass** der Pressstempel (36) sich während des Aufheizens des Haufwerks von Glaspartikeln (32) um ein vorgegebenes Maß bewegt, das insbesondere weniger als 1 mm und besonders bevorzugt zwischen 0,3 und 0,5 mm beträgt.

**18.** Verfahren zum Betrieb eines Ofens basierend auf einer Steuervorrichtung für die Steuerung eines Pressvorgangs, wobei ein Druck-, Weg- und/oder Geschwindigkeits-sensor mindestens einen Druck-, Orts-, und/oder Bewegungsparameter eines Pressstempels (36) erfasst, und wobei die Steuervorrichtung den Pressvorgang basierend auf einem Ausgangssignal des Sensors steuert, wobei beim Pressvorgang der Pressstempel (36) - gegebenenfalls über einen zwischengeschalteten Presskolben (28) - auf ein Haufwerk von Glaspartikeln (32) wirkt und Druck ausübt, welches Haufwerk von Glaspartikeln (32) in einen Presskanal (30) einer Muffel (24) eingeführt ist, und wobei das Auslösekriterium für die Prozesssteuerung eine über den Sensor erfasste Änderung mindestens eines Bewegungsparameters des Pressstempels (36), entsprechend der Erweichung des Haufwerks von Glaspartikeln (32), ist.

**19.** Verfahren nach Anspruch 12, **dadurch gekennzeichnet, dass** nach der Beendigung von Mikroimpulsen (60, 62, 64, 66, 68, 70) des Bewegungsparameters das Haufwerk von Glaspartikeln (32) innerhalb zehn Minuten, insbesondere innerhalb fünf Minuten, bevorzugt innerhalb drei Minuten auf eine vorgegebene Temperatur erhitzt wird, welche mindestens der dilatometrischen Erweichung des Haufwerks von Glaspartikeln entspricht.

**20.** Verfahren nach dem Anspruch 18 oder 19, **dadurch gekennzeichnet,**

- **dass** nach dem Pressvorgang ein Kristallisationsprogramm gestartet wird, bei dem der Pressstempel (36) keinen Druck mehr ausübt,

**21.** Verfahren nach einem der Ansprüche 18 bis 20, **dadurch gekennzeichnet, dass** im Temperaturenbereich zwischen dem Transformationsbereich bzw. Transformationspunkt Tg des Haufwerks von Glaspartikeln und 100 °C Ober den Transformationsbereich Tg (Tg + 100 °C), insbesondere zwischen 30 °C über den Transformationsbereich Tg (Tg + 30 °C) und 80 °C über den Transformationsbereich Tg (Tg + 80 °C), eine Keimbildung des Haufwerks von Glaspartikeln (32) stattfindet.

**22.** Verfahren nach einem der Ansprüche 18 bis 21, **dadurch gekennzeichnet, dass** nach dem Pressvorgang und/oder nach dem Kristallisationsprogramm ein Abkühlprozess und/oder ein Entformungsprozess gestartet wird bzw. werden, wobei insbesondere eine gipsbasierte dentale Einbettmasse In einer Muffel (24) verwendet wird.

**23.** Verfahren nach einem der Ansprüche 18 bis 22, **dadurch gekennzeichnet, dass** am Ende des Verfahrens sich ein Block aus Lithiummetasillkat und/oder aus Lithiumsilikat mit Keimbildnern ergibt.

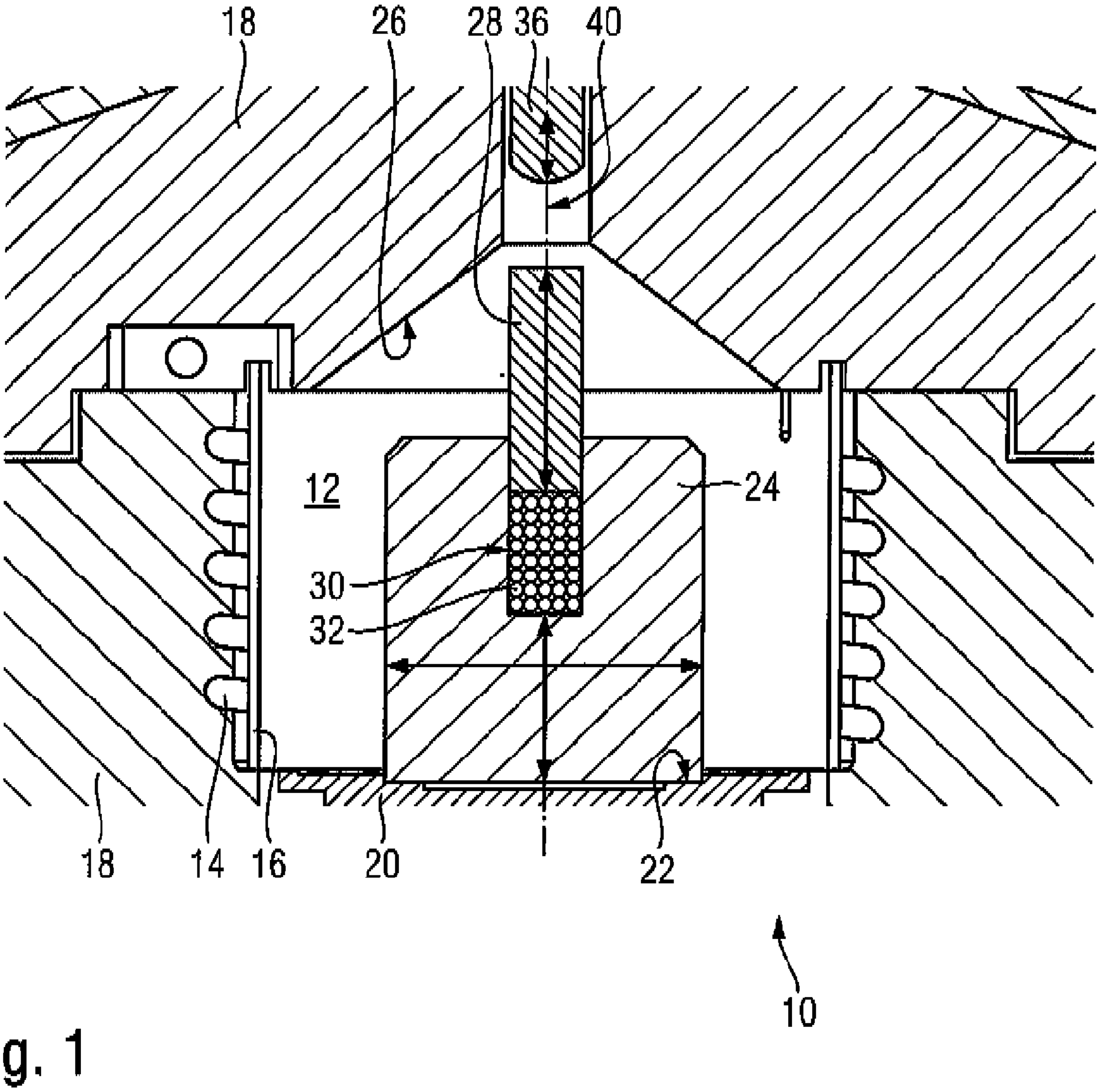


Fig. 1

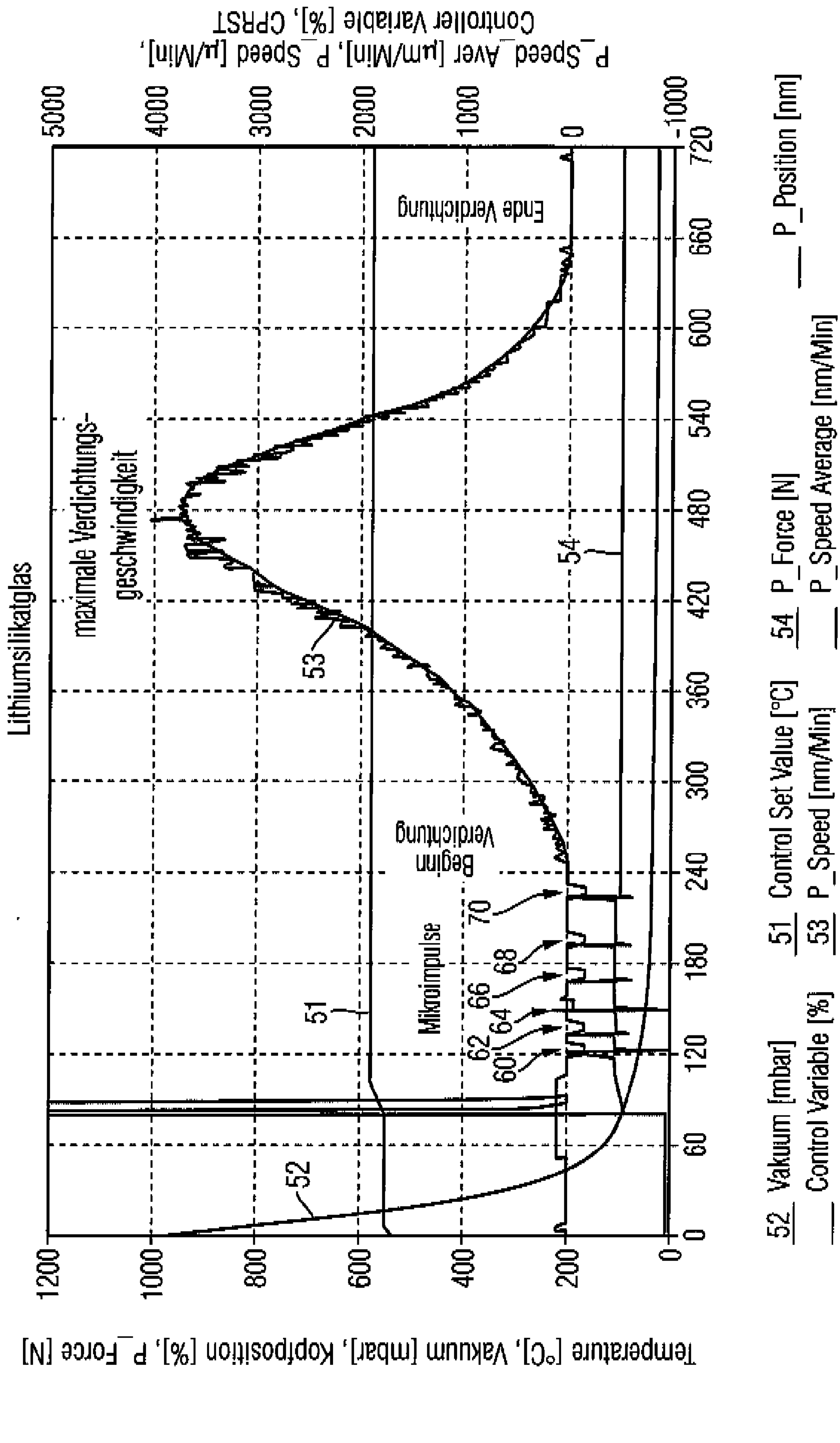
Lithiumsilikatglas
maximale Verdichtungs-geschwindigkeit
Beginn Verdichtung
Ende Verdichtung
Mikroimpulse
Temperature [°C], Vakuum [mbar], Kopfposition [%], P_Force [N]
P_Speed_Aver [µm/Min], P_Speed [µ/Min], Controller Variable [%], CPRST
52 Vakuum [mbar]
51 Control Set Value [°C]
54 P_Force [N]
P_Position [nm]
Control Variable [%]
53 P_Speed [nm/Min]
P_Speed Average [nm/Min]
51
52
53
54
60
62
64
66
68
70
1200
1000
800
600
400
200
0
5000
4000
3000
2000
1000
0
-1000
0
60
120
180
240
300
360
420
480
540
600
660
720

Fig. 2

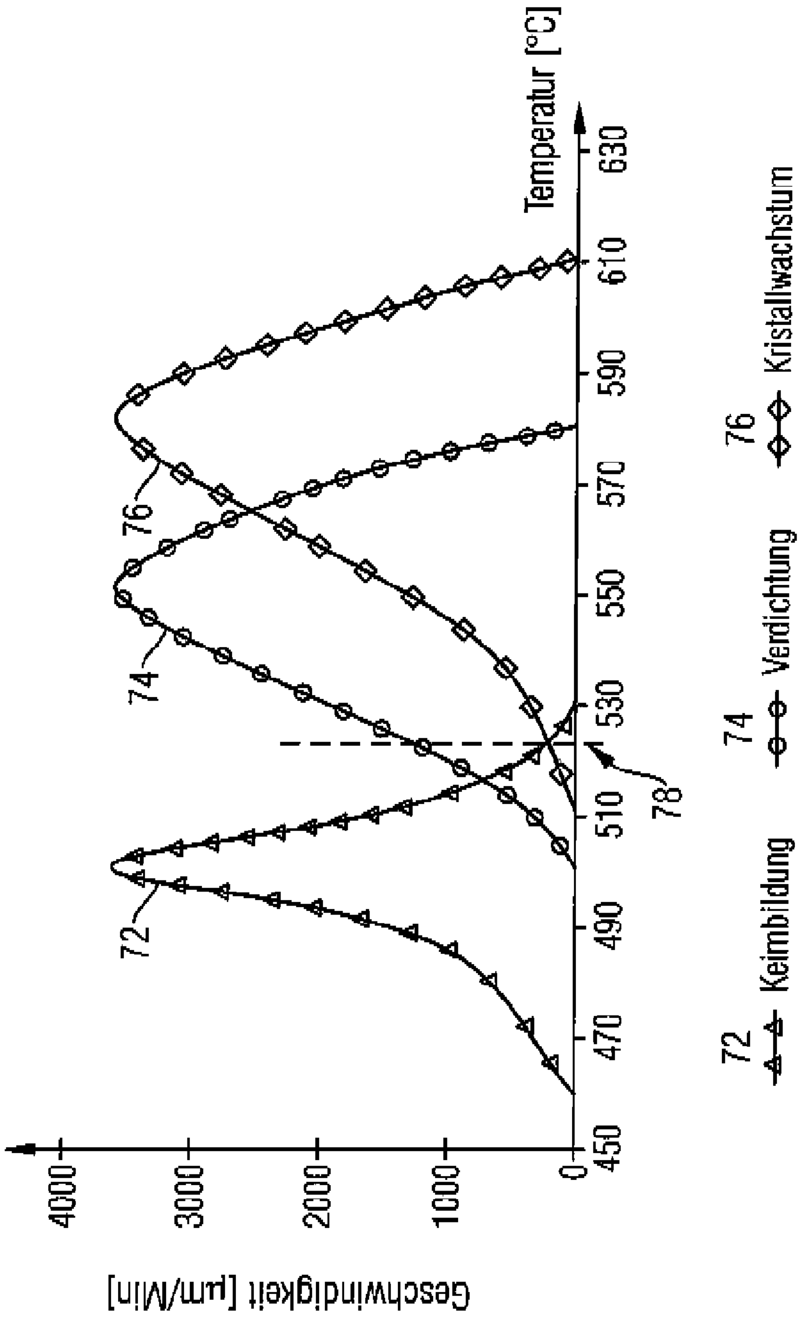
Geschwindigkeit [µm/Min]
4000
3000
2000
1000
0
450
470
490
510
530
550
570
590
610
630
Temperatur [°C]
72
74
76
78
72 Keimbildung
74 Verdichtung
76 Kristallwachstum

Fig. 3

Europäisches Patentamt
European Patent Office
Office européen des brevets

# EUROPÄISCHER RECHERCHENBERICHT

Nummer der Anmeldung
EP 16 19 8859

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | EP 1 915 973 A1 (IVOCLAR VIVADENT AG [LI]) 30. April 2008 (2008-04-30) * Spalte 43 - Spalte 44; Ansprüche 1-3,10-12; Abbildungen 1-2 * ----- | 1-15 | INV. F27B17/02 |
| X | EP 0 438 802 A1 (IVOCLAR AG [LI]) 31. Juli 1991 (1991-07-31) * Spalte 2, Zeile 32 - Spalte 3, Zeile 56; Abbildungen 4,5 * * Spalte 5, Zeile 55 - Spalte 6, Zeile 55; Ansprüche 1,11 * ----- | 1,12 | |
| X | EP 1 978 321 A1 (IVOCLAR VIVADENT AG [LI]) 8. Oktober 2008 (2008-10-08) * Absatz [0015] - Absatz [0021]; Ansprüche 1-3 * ----- | 1,12 | |
| X | EP 2 886 080 A1 (IVOCLAR VIVADENT AG [LI]) 24. Juni 2015 (2015-06-24) * Absätze [0032], [0038], [0052]; Ansprüche 1-8 * ----- | 1,12 | RECHERCHIERTE SACHGEBIETE (IPC) |
| X | US 2013/302459 A1 (MILLER STEPHAN [DE]) 14. November 2013 (2013-11-14) * Ansprüche 1-18 * ----- | 1,12 | F27B F27D |
| E | EP 3 096 100 A1 (IVOCLAR VIVADENT AG [LI]) 23. November 2016 (2016-11-23) * Ansprüche 1-14; Abbildungen 1-5 * ----- | 1-15 | |

Der vorliegende Recherchenbericht wurde für alle Patentansprüche erstellt

1

| Recherchenort | Abschlußdatum der Recherche | Prüfer |
|---|---|---|
| München | 25. April 2017 | Gavriliu, Alexandru |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument

EPO FORM 1503 03.82 (P04C03)

## ANHANG ZUM EUROPÄISCHEN RECHERCHENBERICHT ÜBER DIE EUROPÄISCHE PATENTANMELDUNG NR.

EP 16 19 8859

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

25-04-2017

| Im Recherchenbericht angeführtes Patentdokument | Datum der Veröffentlichung | Mitglied(er) der Patentfamilie | Datum der Veröffentlichung |
|---|---|---|---|
| EP 1915973 A1 | 30-04-2008 | AT 460133 T | 15-03-2010 |
| | | DE 102006050830 A1 | 30-04-2008 |
| | | EP 1915973 A1 | 30-04-2008 |
| | | JP 4902493 B2 | 21-03-2012 |
| | | JP 2008132323 A | 12-06-2008 |
| | | US 2008099939 A1 | 01-05-2008 |
| EP 0438802 A1 | 31-07-1991 | AU 617064 B2 | 14-11-1991 |
| | | CA 2034893 A1 | 27-07-1991 |
| | | DE 4002358 C1 | 24-10-1991 |
| | | EP 0438802 A1 | 31-07-1991 |
| EP 1978321 A1 | 08-10-2008 | DE 102007015435 A1 | 02-10-2008 |
| | | EP 1978321 A1 | 08-10-2008 |
| | | JP 4917565 B2 | 18-04-2012 |
| | | JP 2008253770 A | 23-10-2008 |
| | | US 2008237211 A1 | 02-10-2008 |
| EP 2886080 A1 | 24-06-2015 | CN 105658173 A | 08-06-2016 |
| | | EP 2886080 A1 | 24-06-2015 |
| | | JP 2017500140 A | 05-01-2017 |
| | | KR 20160100293 A | 23-08-2016 |
| | | US 2016184062 A1 | 30-06-2016 |
| | | WO 2015091779 A1 | 25-06-2015 |
| US 2013302459 A1 | 14-11-2013 | DE 102010053873 A1 | 14-06-2012 |
| | | EP 2627281 A1 | 21-08-2013 |
| | | US 2013302459 A1 | 14-11-2013 |
| | | WO 2012076134 A1 | 14-06-2012 |
| EP 3096100 A1 | 23-11-2016 | EP 3096100 A1 | 23-11-2016 |
| | | US 2016338805 A1 | 24-11-2016 |
| | | WO 2016188901 A1 | 01-12-2016 |

EPO FORM P0461

Für nähere Einzelheiten zu diesem Anhang : siehe Amtsblatt des Europäischen Patentamts, Nr.12/82

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 2014131588 A1